# EUROPEAN PATENT APPLICATION

(11) **EP 2 878 286 A1**
(43) Date of publication of application: **03.06.2015**
(21) Application number: 13822666.7
(22) Date of filing: 01.07.2013
(51) Int. Cl.: A61F 5/455, A61F 13/472

(54) **PORTABLE CONTAINER FOR HOLDING LIQUIDS AND ALSO FOR URINATION, ESPECIALLY FOR FEMALES**

(30) Priority: 27.07.2012 BR 12018873
(71) Applicant: Boabaid, Flavio Marçal, 88015-420 Florianópolis - SC (BR)
(72) Inventor: Boabaid, Flavio Marçal, 88015-420 Florianópolis - SC (BR)
(74) Representative: Dall'Olio, Christian
(86) International application number: PCT/BR2013/000244
(87) International publication number: WO 2014/015399

(57) **Abstract**

Portable container for containment of urination fluids, which comprises a portable U-shaped container (5), bag or reservoir for containing human bodily fluids, especially urine, especially for female urination. The container (5) is endowed with walls (8) that form an internal (6) or external (8) compartment for keeping wipes or others, internally coated with an absorbent material and externally with a waterproof fabric, so that once the container is sealed (5) via the folding of the superior-longitudinal flap (2), the device (5) is portable for the necessary time or for the transportation of the material contained until its disposal.

## Description

### INTRODUCTION

This specification describes enhancements constructive in nature, thus proposed to improve the containment and transportation of fluids, including in healthcare environments, such as, for example, clinical analysis laboratories, hospitals and outpatient clinics, as well as the urination itself, especially among women, particularly on overcrowded sites or where, for whatever reason, the presence of dirt is likely both on sanitary installations and in the places where they are installed.

Thus presented, is a portable container that may be converted into a bag for the transportation of fluids including urination substance, in which the material, biological or non-biological, is contained in the constructive settings thereby considered, by the action of the absorbent material that lines the inner walls of the container and by the waterproof coating provided on its outer walls.

A first constructive solution is the provision of a superior-longitudinal edge, whose face meeting the outer wall of the device is adhesive, or adherent in any form, functioning as a means of sealing off the container.

In a second constructive option, the device is provided with, in addition to the superior-longitudinal flap that is adhesive or adherent in any form, an internal receptacle for the packing of paper tissues or another sanitizing material, provided on one or both inner walls; and in yet a third possible solution, this receptacle is provided on one or both outer walls.

In any case, the tissue or substitute material may or may not be used by the user, and once the portion is superimposed or the portions adherent to the adjacent wall of the container are superimposed, it is discarded.

The portable container thus designed is able to allow both the containment and transportation of fluids, in relation to urination, especially in women, in a safe and discreet fashion that is free of dirt common to appliances and public health facilities in general, providing comfort, especially in places of overcrowding or greater demand, such as, for example, at exhibitions, art events and large shopping centers.

### STATE OF THE ART

Devices that somehow relate to the containment of fluids and especially to female urination are few but relatively widespread in common practice.

Nonetheless, such devices do not obviate successive installments in view of their portability, or exist solely to prevent dermal contact with the sanitary apparatus, and, for the transportation of fluids itself in healthcare environments, solely contemplate the function itself and the fact that they are molded in flexible material.

In research carried out on the state of the art, as related below, it was possible to find documents involving various operating concepts, with those whose operation followed dissimilar concepts and not related to the present specification being discarded, it was possible to select the following:

MU8400036-8 U, with submission date of 3/2/2004, under title: DISPOSABLE EXTENSOR DEVICE FOR FEMALE URINATION, which comprises a constructive provision that prevents contact of the woman with the bowl, acting as a fluid dispenser in relation to its the primary use, and endowed with an inner compartment containing wipes for hygiene.

MU8600376-3 U, with submission date of 3/21/2006, under title: AUXILIARY FEMALE URINAL DEVICE WITH AUTOMATIC SAFETY FLAP, which comprises a cone of biodegradable paper that fits the female anatomy with only two folding movements.

PI0700316-1 A, with submission date of 1/17/2007, under title: DISPOSABLE DEVICE FOR FEMALE URINATION, whose practical effect allows urination to be performed while standing.

MU8702065-3 U2, with submission date of 12/20/2007, under title: DISPOSABLE TOILET FUNNEL FOR FEMALE URINE, which adapts to the woman's anatomy and allows urination without contact with the toilet bowl.

MU8902088-0 U2, with submission date of 9/1/2009, under title: DISPOSABLE OBJECT FOR INNER COMFORT, which is actually a plastic bag open at the top edge and which allows the retention of the contents inside. Lower down there is an opening where the material is discarded. The operating concept of portability is given due to the various folds of the object.

US 12/769,775, with submission date of 4/29/2010, under title: SET OF DISPOSABLE ABSORBENT SANITARY ARTICLES INCLUDING AN INTEGRATED DISPOSAL SHEET, which is in fact an absorbent assembly structured to allow the disposal sheets to be separated manually.

MU9000737-9 U2, with submission date of 5/26/2010, under title: FAN-SHAPED URINARY FUNNEL, in which a fan-shaped funnel is claimed, whose function consists in releasing the folding and/or detachable flap containing an absorbent paper or intimate absorbent on its inner side. The fitting of the lower left side joins a cut on the lower right side, setting the conical projection of the funnel.

Of the known documents related to the state of the art, it appears that, although some of them contain operational concepts related to urination, the inference is that practically all have the primary purpose of avoiding contact of the dermis with the sanitary apparatus, via facilitating urination while standing, or at least at a distance of no contact.

To speak of container configuration and not the conductive duct is unusual, with the exception of document MU8902088-0 U2 which, nonetheless, contemplates in practice, as shown by the illustrative examples, a portable toilet, including the object for sitting upon in an equivalent format, or at least maintaining it in the corresponding position. The upper opening, for example, is sufficiently large for the user to defecate.

On the other hand, while document PI1101742-2 A2 makes clear reference to female absorbents, documents MU8702065-3 U2 PI10700316-1 A MU8600376-3 U and U MU8400036-8 provide equivalent solutions that are extremely similar among themselves. All these relate to the mechanics of directing the urine, whether for women to urinate whilst standing, or in order to avoid their contact with the sanitary apparatus.

The latter presents a compartment for sanitizing wipes, but claims it inside the urination device, without the ability to see it on the outer wall, for example

That said, it is observed that the related documents, although referring to solutions for female urination, contemplate them through the prism of the difficulty of access to conventional toilets, even proposing urination whilst standing or at least at a distance from the sanitary apparatus. The only exception, as related above, is document MU8902088-0 U2, which, in differentiation from the object proposed above, comprises a portable sanitary bowl, whilst considering the traditional plastic conformation of this apparatus.

Therefore, in the case of the improvements proposed by means of this specification, there are significant operational improvements, firstly because, when used as a urinal device they do not have the power to impose urination whilst standing among women and, secondly, because the main purpose is neither the direction of urinary fluid, neither to resemble a toilet itself, where even defecation is permitted, but rather to provide an apparatus able to contain urine for the necessary time until the container can be disposed of at an appropriate site, as well as to provide the minimum sanitization required via wipes or substitutes that may be internally or externally provided, i.e. on the inner face or the outer face of one of or both side walls, which, incidentally, are coated internally with absorbent material and externally covered with waterproof fabric.

Finally, it must be noted that the transportation of the material contained assumes the seal provided by the superior-longitudinal flap, in turn provided with edge or edges adherent to the outer wall with which they are overlapping, whether used as a urinal or as a fluid container for healthcare purposes.

### SUMMARY OF OBJECT OF PATENT

The account of the relevant state of the art as described above and below, is taken as the subject of this constructive solution specification which, applied to the portable (5) container, is able to convert it into an auxiliary device for especially female urination, and via efficient means for containing and transporting fluids for healthcare purposes.

In fact, it relates to a collecting (5) bag internally equipped with walls (6) with absorbent coating, whose plastic configuration is preferential in a *U-shape* precisely so that there are no corners and so that the risk of leakage is virtually eliminated even though production is not by injection.

Externally, the aforementioned bag (5) is coated with waterproof material (7), and the superior-longitudinal (2) flap is endowed with an adhesive, auto-adhesive portion (1) or portions, for example, which encourage the sealing of the bag (5) once the flap is superimposed (2) in relation to the outer face of the adjacent (3) wall.

Internally, the device is equipped with material (6) able to absorb the urine fluid immediately, so that once the container is sealed (5) by folding the superior-longitudinal (2) adhesive flap against the opposite (3) wall, it is safely contained.

Wipes or a substitute sanitizing material are provided in the compartment (9) or compartments present both on the inner face (6) and the outer face (7) of one or both side walls (8).

### DESCRIPTION OF FIGURES

The characterization of the improvements under discussion is given by means of representative illustrations of the proposed constructive solution, so that both the main body preferably U-shaped, and the projection of the superior-longitudinal flap and the preferential wall formation can be seen clearly enough.

The figures express the preferred way of completing the idealized product, and underlie the present description by means of numerical and consecutive remissions that clarify constructive and operative aspects perhaps implied in the representation adopted, precisely determining the tutelage postulated. These figures are merely illustrative and may display variations, provided that they do not deviate from that initially pledged.

Thus note that:

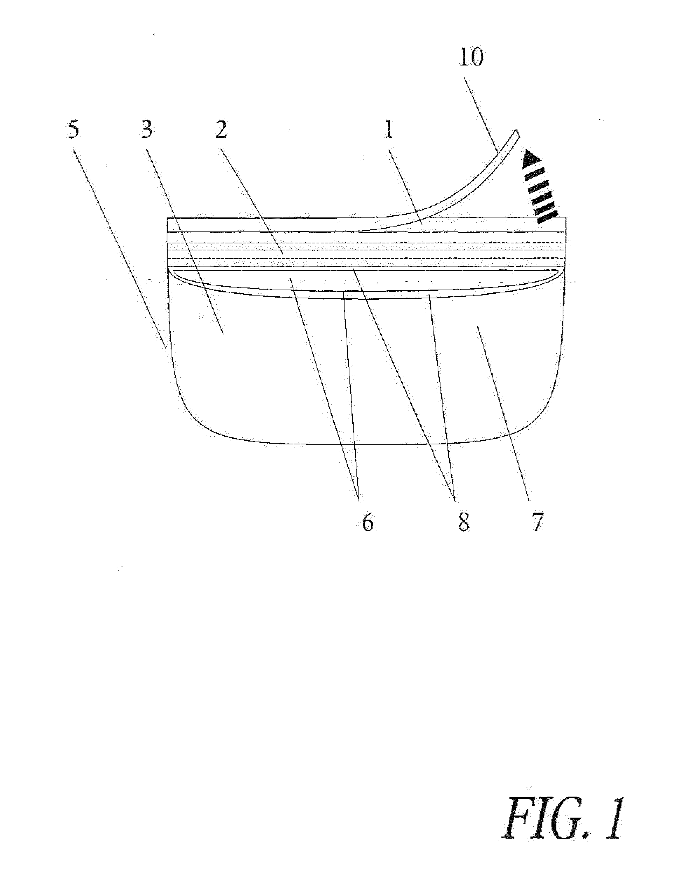

- FIGURE 1 in previous view, describes the constructive solution in which the adhesive (1) edge provided along the superior-longitudinal flap (2) is provided with an adhesive portion which, meeting the opposite wall (3) when folding the flap (2) encourages the sealing of the container (5). The same figure allows the visualization of the start-up direction of the protective tape (10) of the adhesive portion provided at the edge (1) and the inner walls (8), especially the thick absorbent coating (6), and also the waterproof fabric or material that covers the outside (7).

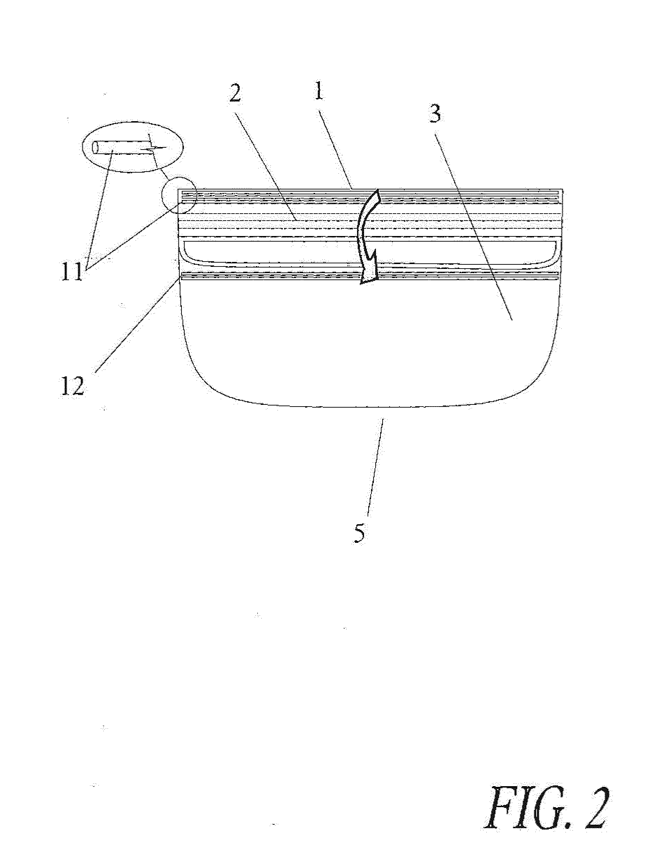

- FIGURE 2 also in previous view, describes the constructive solution in which the adhesive (1) edge provided along the superior-longitudinal flap (2) is provided with threads (11) that fit into the opposing threads (12) provided on the wall (3) when folding the flap (2) encouraging the sealing of the container (5). The figure offers a detailed view of the threads (11) whose constructivity is the same as that of the opposing (12) threads.

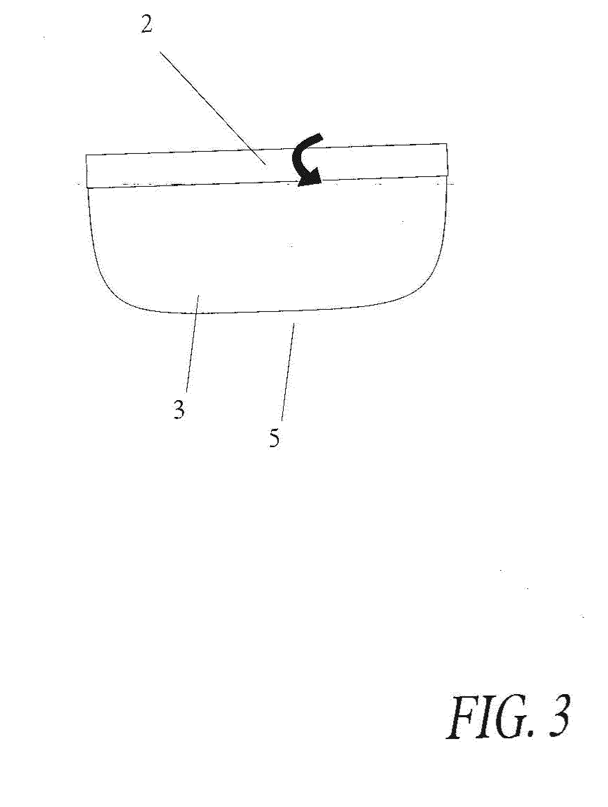

- FIGURE 3 from the same previous views, describes the closed (5) container, i.e. with the superior-longitudinal adhesive flap (2) superimposed on the opposite wall (3).

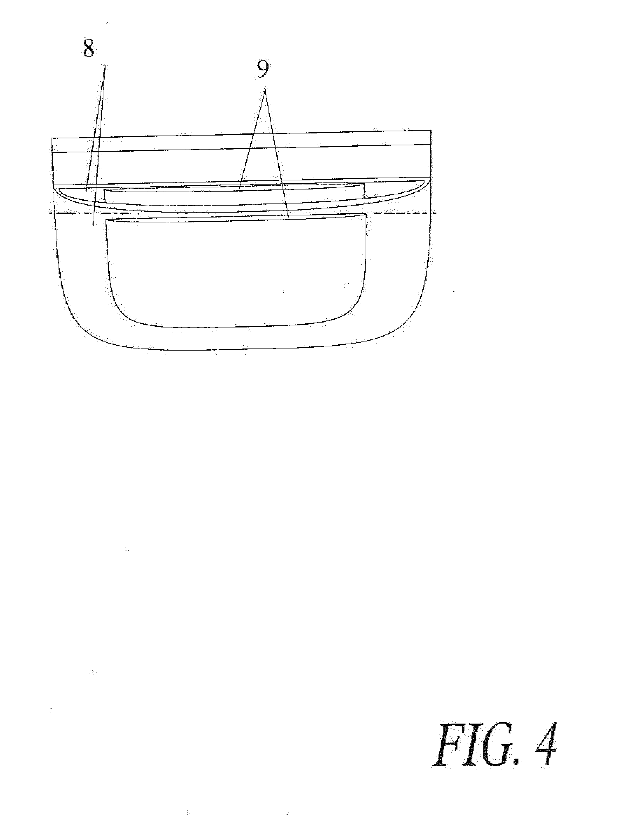

- FIGURE 4 still in previous view, depicts the container (5) informing the possible positioning for one or more compartments (9) for wipes or a replacement sanitizing material, which may be installed on either one of the inner walls (8) or on one of both of the external walls (8).

### DETAILED DESCRIPTION OF OBJECT OF PATENT

The object of this specification consists of constructive improvements resulting in a container specially designed to work as a device for aiding female urination, although there are no technical or operational impediments preventing it from being used by males also.

Moreover, it is also a container which can be used in the containment and transportation of fluid or material for healthcare purposes such as, for example, blood, blood tissue, fluid or inter-cellular substances.

In the case of use as a urinal, the main purpose is to provide urination in places where access to the toilet is difficult or undesirable, i.e. where the demand is too great, or where hygiene conditions are not the best.

Thus, the improvements related prescribe a container, preferably U-shaped, whose superior-longitudinal flap functions as a sealing element, since it is provided with a self-adhesive edge or system, or a substitute sealing mechanism either by pressure, or by closing in the case of the use of zippers or similar means, or superimposing, in the case of use of connecting hooks and loops commonly known by the term Velcro.

Furthermore, the container is also endowed with a compartment for wipes or a substitute sanitizer, which may be provided on one or both walls, externally or internally.

Finally, the walls of the container are lined internally with a thick absorbent material to contain storable liquid, via the seal provided by the folding of the superior-longitudinal flap against the opposite wall; and externally with waterproof fabric, so that the container is portable and used even after the necessary time so that it is disposed of properly.

## Claims

1. Portable container for containment of urination fluids, comprising a preferably u-shaped bag endowed with a superior-longitudinal (2) flap foldable against the opposite wall (3), **characterized by** the fact that the superior-longitudinal flap (2) allows the sealing of the container (5) by superimposing the edge (1) in relation to the opposite (3) wall.

2. Portable container for containment of urination fluids, in accordance with claim no.1, **characterized by** the fact that the edge (1) provided along the superior-longitudinal (2) flap may be resolved with the provision of an adhesive portion (1) protected by flat (10) start-up tape.

3. Portable container for containment of urination fluids, in accordance with claim no.1, **characterized by** the fact that the edge (1) provided along the superior-longitudinal (2) flap may be resolved with rigid filaments (11), or a substitute mechanism, adjacent with their pairs (12) provided on the opposite wall (3) when the edge is superimposed (1).

4. Portable container for containment of urination fluids, in accordance with the previous claims, **characterized by** the provision, in healthcare environments, of a container for the containment and transportation of fluids.

5. Portable container for containment of urination fluids, in accordance with the previous claims, **characterized by** the fact that the side walls (8) that configure the container (5) are internally coated with an absorbent material (6), sufficiently thick for fluid to be contained, and externally with waterproof fabric (7).

6. Portable container for containment of urination fluids, in accordance with claim no. 5, **characterized by** the fact that, on one or both of the walls (8), internally (6) or externally (7), a compartment is endowed (9) for the conditioning of wipes or substitute sanitizers.
